# EUROPEAN PATENT APPLICATION

(11) **EP 1 441 221 A1**
(43) Date of publication of application: **28.07.2004**
(21) Application number: 04001205.6
(22) Date of filing: 21.01.2004
(51) Int. Cl.: G01N 27/64, B64G 1/10

(54) **Method and device for observing the atmosphere within an altitude range of about 100 km - 1000 km**

(30) Priority: 24.01.2003 JP 2003015829
(71) Applicant: Japan Aerospace Exploration Agency, Chofu City, Tokyo (JP)
(72) Inventor: Kuninaka, Hitoshi, Kawasaki City Kanagawa Pref. (JP); Yamagiwa, Yoshiki, Hamamatsu City Shizuoka Perf. (JP)
(74) Representative: von Hellfeld, Axel, Dr. Dipl.-Phys.

(57) **Abstract**

Ion particles are discharged so as to be trapped with magnetic field lines of the earth, and collided with high-altitude neutral air to generate high velocity neutral particles through charge exchange. The high velocity neutral particles are trapped. In this case, the distance to the high-altitude neutral air from at least one of the discharging positions of the ion particles and the trapping positions of the high velocity neutral particles is determined based on the period of time between the discharging timings of the ion particles and the trapping timings of the high velocity neutral particles. Moreover, the direction of the high-altitude neutral air is determined based on the trapping direction of the high velocity neutral particles. In addition, the space position of the high-altitude neutral air is determined based on the trapping direction of the high velocity neutral particles.

## Description

### BACKGROUND OF THE INVENTION

### Field of the Invention

This invention relates to a method for observing high-altitude neutral air and a device for observing high-altitude neutral air which are usable in space operational business enterprise and space weather forecast business enterprise.

### Description of the related art

As has gotten a lot of attention in the falling of the Mir space station at March, 2001, if a large-scaled space structure is plunged into atmosphere and reached to earth, it is concerned that the space structure affects on our social life to some degree. In this point of view, data concerning high-altitude neutral air are very important because the orbit altitude of a space satellite may be decreased due to the atmosphere drag in the high-altitude neutral air and the plunge timing of the space satellite into atmosphere can be predicted from the high-altitude neutral air data.

In a conventional observation for the high-altitude neutral air, an observing instrument is mounted on a space satellite, which is disposed in the high-altitude neutral air. As a result, since the observing instrument is positioned in a given area of the high-altitude neutral air, the observation for the high-altitude neutral air is carried out at every area where the observing instrument is positioned.

Since the observing area is contaminated by positioning the space satellite, with the conventional technique, the high-altitude neutral air can not be observed precisely. Moreover, with the conventional technique, only the data concerning a given area of the high-altitude neutral air by positioning the observing instrument in the given area can be obtained, so the total data concerning the high-altitude neutral air can not be obtained simultaneously.

The data concerning the high-altitude neutral air can be obtained from the Jacchia model (Standard Jacchia Reference Atmosphere 1977) which is a simulated and modeled distribution of high-altitude neutral air on the changes in altitude of many space satellites launched in the past. Since the predictive accuracy of the Jacchia model is poor, however, it can not be employed in a technical field requiring prompt response and accuracy such as the prediction in atmosphere plunge of a space satellite and the like.

### SUMMERY OF THE INVENTION

It is an object of the present invention, in this point of view, to observe the high-altitude neutral air widely and precisely.

In order to achieve the above-mentioned objects, this invention relates to a method for observing high-altitude neutral air, comprising the steps of:
discharging ion particles so as to be trapped with magnetic field lines of the earth,
colliding the ion particles with high-altitude neutral air to generate high velocity neutral particles through charge exchange, and
trapping the high velocity neutral particles to determine the distance to the high-altitude neutral air from at least one of the discharging positions of the ion particles and the trapping positions of the high velocity neutral particles on the period of time between the discharging timings of the ion particles and the trapping timings of the high velocity neutral particles, to determine the direction of the high-altitude neutral air on the trapping direction of the high velocity neutral particles, and to determine the space position of the high-altitude neutral air.

In the present invention, for example, a given ion source is disposed on the orbit of the earth, and then, ion particles are discharged from the ion source so as to be trapped by the magnetic field lines of the earth. When the ion particles are collided with the high-altitude neutral air, high velocity neutral particles are generated through the charge exchange with the ion particles in the high-altitude neutral air. The neutral particles travel inertially without the disturbance of the magnetic field lines of the earth, and trapped with a given neutral particle analyzer disposed on the orbit of the earth.

The discharging velocities of the ion particles can be predetermined, and the velocities of the neutral particles can be measured with the neutral particle analyzer. Moreover, the relative position between the ion source and the neutral particle analyzer can be predetermined, and the discharging angles of the ion particles from the ion source and the observing angle of the neutral particle analyzer can be predetermined. Therefore, if the periods of time between the discharging timings of the ion particles and the trapping timings of the neutral particles are measured, at least one of the distances between the high-altitude neutral air and the ion source and between the high-altitude neutral air and the neutral particle analyzer can be determined.

Moreover, since the neutral particles can be trapped with the neutral particle analyzer, the direction of the high-altitude neutral air can be determined from the trapping directions of the neutral particles.

In addition, in the present invention, since the distance for the high-altitude neutral air and the direction of the high-altitude neutral air are measured as mentioned above, the space position of the high-altitude neutral air can be determined therefrom.

Herein, the wording "high-altitude neutral air" means an atmosphere within an altitude range of about 100km-1000km.

Also, the wording "charge exchange" means a reaction where an ion particle "A" is collided with an ion particle "B", causing the charge transfer of the ion particle "A" to the ion particle "B" and thus, generating a high velocity neutral particle "A" and a high velocity neutral particle "B" (A*+B → A+B*).

In the present invention, since the high velocity neutral particles can be generated from the charge exchange with the ion particles in the high-altitude neutral air, if the trapping frequency of the neutral particles is measured with the neutral particle analyzer, the particle density of the high-altitude neutral density can be determined.

If the ion particles are made of the same particles as the neutral particles, the energies of the ion particles and the energies of the neutral particles can be conserved before and after the collision between the ion particles and the neutral particles. Therefore, the kinetic energies of the neutral particles are equal to the kinetic energies of the ion particles. In contrast, if the ion particles are made of different particles from the neutral particles, the kinetic energies of the neutral particles are increased and decreased on the differences in ionization voltage between the ion particles and the neutral particles before and after the collision therebetween.

Since the sorts of the ion particles are known, if the increasing and decreasing in kinetic energy of the ion particles are measured, the sorts of particles in the high-altitude neutral air can be determined through the collision, and thus, the composition of the high-altitude neutral air can be determined.

It is desired that the ion particles are made of particles which rarely exist on the high and low orbits of the earth. In this case, the ion particles can be recognized clearly against other particles in the space. Concretely, the ion particles may be made of krypton particles or xenon particles.

The ion particles may be discharged in pulse or modulation. In this case, the discharging timings of the ion particles and the trapping timings of the high velocity neutral particles can be recognized clearly, and the distance for the high-altitude neutral air can be measured easily and precisely.

In this way, according to the present invention, the space position, the density and the composition of the high-altitude neutral air can be determined precisely. If the discharging angles of the ion particles from the ion source and the observing angle of the neutral particle analyzer are controlled appropriately, the density and the composition of the high-altitude neutral air can be widely determined in a short period of time.

### BRIEF DESCRIPTION OF THE DRAWINGS

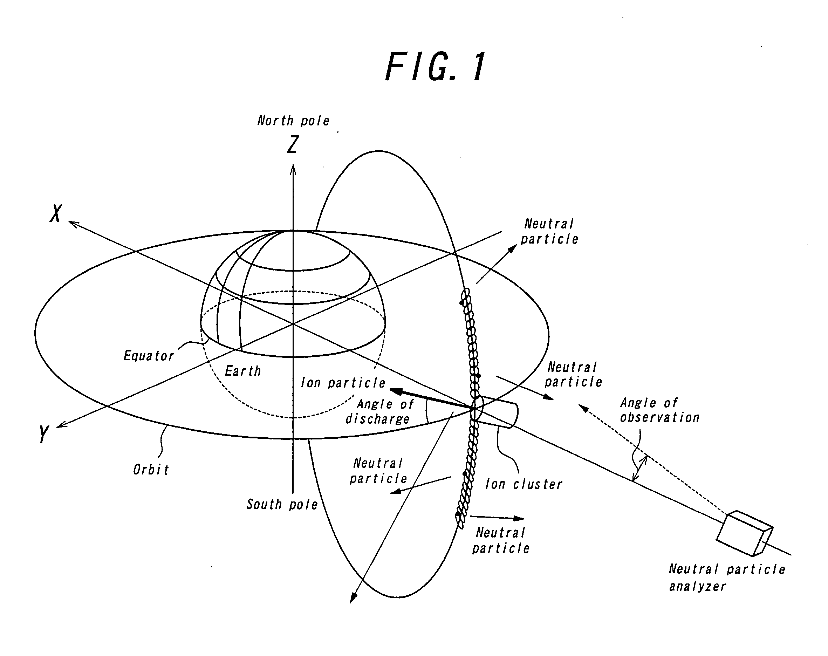

For better understanding of the present invention, reference is made to the attached drawings, wherein
Fig. 1 is an explanatory view relating to a method for observing high-altitude neutral air according to the present invention.

### DESCRIPTION OF THE PREFERRED EMBODIMENTS

This invention will be described in detail with reference to the accompanying drawings.
Fig. 1 is an explanatory view relating to a method for observing high-altitude neutral air according to the present invention.
In Fig. 1, an ion cluster source is disposed as an ion source on the orbit above the equator of the earth, and a neutral particle analyzer is disposed in the rear side of the ion cluster source. Ion particles are discharged from the ion cluster source, and trapped with the magnetic field lines generated from the axis of the earth. In this case, the ion particles are rotated along the magnetic field lines, which is defined as "Larmor motion", and moved north and south. If a given condition is satisfied, the mirror confining mechanism is generated, so that the ion particles are moved repeatedly north and south.

In this case, the ion particles are collided with inner particles in the high-altitude neutral air (not shown) at the black points in Fig. 1, causing the charge transfer of the ion particles to the inner particles and thus, generating neutral particles in the directions designated by the arrows. The neutral particles travel inertially at their respective high velocities without the disturbance of the magnetic field lines, and trapped and detected with the neutral particle analyzer.

The discharging velocities of the ion particles can be predetermined, and the velocities of the neutral particles can be measured with the neutral particle analyzer. On the other hand, the relative position between the ion cluster source and the neutral particle analyzer can be predetermined. In addition, the discharging angles of the ion particles from the ion cluster source can be predetermined and the observing angle of the neutral particle analyzer can be predetermined. Therefore, if the periods of time between the discharging timings of the ion particles and the trapping timings of the neutral particles are measured, at least one of the distances between the high-altitude neutral air and the ion cluster source and between the high-altitude neutral air and the neutral particle analyzer can be determined.

The direction of the high-altitude neutral air can be determined from the trapping directions of the neutral particles with the neutral particle analyzer.

As mentioned above, it is desired that the ion particles are made of particles which rarely exist on the orbit of the earth such as krypton particles or xenon particles in order to be distinguished from other particles in nature. In order to enhance the easiness and precision of the measurement of the distance for the high-altitude neutral air, the ion particles may be discharged in pulse or modulation.

If the trapping frequency of the neutral particles are measured with the neutral particle analyzer, the particle density of the high-altitude neutral air can be determined because the high velocity neutral particles can be generated through the charge exchange with the ion particles in the high-altitude neutral air. If the changes in kinetic energy of the neutral particles are measured, the composition of the high-altitude neutral air can be determined because the sorts of the ion particles are known and thus, the changes in kinetic energy of the neutral particles depend on the sorts of particles in the high-altitude neutral air.

In this embodiment, since it is not required that the ion cluster source and the neutral particle analyzer are disposed directly in the high-altitude neutral air, the space position, the density and the composition of the high-altitude neutral air can be determined precisely without the contamination of the ion cluster source, the neutral particle analyzer and the like. Moreover, if the discharging angles of the ion particles from the ion cluster source and the observing angle of the neutral particle analyzer are controlled appropriately, the density and the composition of the high-altitude neutral air can be determined widely in a short period of time.

It may be that the ion cluster source and the neutral particle analyzer can be mounted on a space satellite which is disposed on the orbit of the earth. In this case, if the position of the space satellite is adjusted, the ion cluster source and the neutral particle analyzer can be disposed as illustrated in Fig. 1 to observe the high-altitude neutral air. The ion cluster source and the neutral particle analyzer can be mounted on the same space satellite or respective different space satellites. In the latter case, since the degree of freedom in disposition of the ion cluster source and the neutral particle analyzer can be increased, the high-altitude neutral air can be observed and measured wider.

Although the present invention was described in detail with reference to the above examples, this invention is not limited to the above disclosure and every kind of variation and modification may be made without departing from the scope of the present invention.

As mentioned above, according to the present invention, since it is not required that the ion source and the neutral particle analyzer are disposed directly in the high-altitude neutral air, the space position, the density and the composition of the high-altitude neutral air can be determined precisely. Moreover, when the discharging angles of the ion particles from the ion source and the observing angle of the neutral particle analyzer are controlled appropriately, the density and the composition of the high-altitude neutral air can be determined widely in a short period of time.

## Claims

1. A method for observing high-altitude neutral air, comprising the steps of:
discharging ion particles so as to be trapped with magnetic field lines of the earth,
colliding said ion particles with high-altitude neutral air to generate high velocity neutral particles through charge exchange, and
trapping said high velocity neutral particles to determine the distance to said high-altitude neutral air from at least one of the discharging positions of said ion particles and the trapping positions of said high velocity neutral particles on the period of time between the discharging timings of said ion particles and the trapping timings of said high velocity neutral particles, to determine the direction of said high-altitude neutral air on the trapping direction of said high velocity neutral particles, and to determine the space position of said high-altitude neutral air.

2. The observing method as defined in claim 1, wherein the density of said high-altitude neutral air is determined on the trapping frequency of said high velocity neutral particles.

3. The observing method as defined in claim 1 or 2, wherein the composition of said high-altitude neutral air is determined on the changes in kinetic energy of said high velocity neutral particles for said ion particles.

4. The observing method as defined in any one of claims 1-3, wherein said ion particles are made of krypton particles and/or xenon particles.

5. The observing method as defined in any one of claims 1-4, wherein said ion particles are discharged in pulse.

6. The observing method as defined in any one of claims 1-4, wherein said ion particles are discharged in modulation.

7. A device for observing high-altitude neutral air, comprising:
an ion source disposed on an orbit of the earth, and
a neutral particle analyzer disposed on an orbit of the earth.

8. The observing device as defined in claim 7, wherein said ion source discharges ion particles so as to be trapped with magnetic field lines of the earth.

9. The observing device as defined in claim 8, wherein said ion particles are made of krypton particles and/or xenon particles.

10. The observing device as defined in claim 8 or 9, wherein said ion particles are discharged in pulse.

11. The observing device as defined in claim 8 or 9, wherein said ion particles are discharged in modulation.

12. The observing device as defined in any one of claims 8-11, wherein said neutral particle analyzer traps high velocity neutral particles which are generated from said ion particles through the collision of said ion particles with high-altitude neutral air and the charge exchange with said ion particles.

13. The observing device as defined in any one of claims 7-12, wherein said ion source and said neutral particle analyzer are mounted on the same space satellite.

14. The observing device as defined in any one of claims 7-12, wherein said ion source and said neutral particle analyzer are mounted on respective difference space satellites.
